# EUROPÄISCHE PATENTANMELDUNG

(11) **EP 4 762 936 A1**
(43) Veröffentlichungstag der Anmeldung: **24.06.2026**
(21) Anmeldenummer: 24221656.2
(22) Anmeldetag: 19.12.2024
(51) Int. Cl.: A23C 9/12, C07H 3/06, C12N 9/38, C12P 19/14

(54) **GALACTOOLIGOSACCHARIDE MIT HOHEM LACTULOSEGEHALT**

(71) Anmelder: DMK Deutsches Milchkontor GmbH, 27404 Zeven (DE)
(72) Erfinder: DÖRING, Sven-Rainer, 27404 Zeven (DE); Steffens, Marco, 27404 Elsdorf (DE)
(74) Vertreter: Soilan Rodriguez, Jose David

(57) **Zusammenfassung**

Vorgeschlagen wird ein Galactooligosaccharide, die sich durch einen hohen Lactulosegehalt auszeichnen sowie ein Verfahren zu ihrer Herstellung

## Beschreibung

### GEBIET DER ERFINDUNG

Die Erfindung befindet sich auf dem Gebiet der Lebensmitteltechnologie und betrifft und betrifft Galactooligosaccharide, die sich durch einen hohen Lactulosegehalt auszeichnen sowie ein Verfahren zu ihrer Herstellung.

### TECHNOLOGISCHER HINTERGRUND

Galactooligosaccharide (GOS) 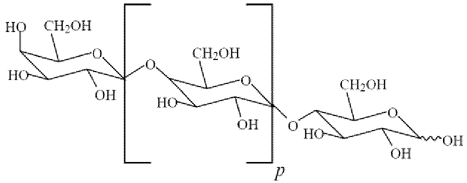 auch bekannt als Oligogalaktosyllactose, Oligogalaktose, Oligolaktose oder Transgalaktooligosaccharide (TOS), gehören zur Gruppe der Präbiotika. GOS kommt in handelsüblichen Produkten wie Nahrung für Säuglinge und Erwachsene vor.

Aufgrund der Konfiguration ihrer glykosidischen Bindungen widerstehen Galactooligosaccharide (GOS) weitgehend der Hydrolyse durch Speichel- und Darmverdauungsenzyme. Galactooligosaccharide werden daher als Präbiotika eingestuft, definiert als unverdauliche Nahrungsbestandteile, die sich vorteilhaft auf den Wirt auswirken, indem sie das Wachstum und/oder die Aktivität nützlicher Bakterien im Dickdarm stimulieren. Die erhöhte Aktivität dieser gesundheitsfördernden Bakterien führt zu einer Reihe von Effekten, sowohl direkt durch die Bakterien selbst als auch indirekt durch die organischen Säuren, die sie durch Fermentation produzieren. Beispiele für Wirkungen sind die Stimulierung der Immunfunktionen, die Aufnahme essentieller Nährstoffe, und die Synthese bestimmter Vitamine.

Galactooligosaccharide sind ein Substrat für Bakterien, wie Bifidobakterien und Laktobazillen. Studien mit Säuglingen und Erwachsenen haben gezeigt, dass mit Galakto-Oligosacchariden angereicherte Lebensmittel oder Getränke zu einer signifikanten Zunahme von Bifidobakterien führen. Diese Zucker kommen natürlich in der menschlichen Milch vor und sind als Oligosaccharide der menschlichen Milch bekannt. Beispiele sind Lakto-N-Tetraose, Lakto-N-Notetraose und Lakto-N-Fucopentaose.

Die menschliche Darmmikrobiota spielt eine Schlüsselrolle im intestinalen Immunsystem. Galacto-Oligosaccharide unterstützen die natürlichen Abwehrkräfte des menschlichen Körpers über die Darmmikroflora, indirekt, indem sie die Anzahl der Bakterien im Darm erhöhen und die Bindung oder das Überleben von *Escherichia coli, Salmonella Typhimurium* und Clostridien hemmen. GOS können das Immunsystem indirekt durch die Produktion von antimikrobiellen Substanzen positiv beeinflussen, indem sie die Vermehrung von pathogenen Bakterien reduzieren. Verstopfung ist ein potenzielles Problem, insbesondere bei Säuglingen, älteren Menschen und schwangeren Frauen. Bei Säuglingen kann die Fütterung mit Säuglingsnahrung mit Verstopfung und hartem Stuhl assoziiert sein. GOS können die Stuhlfrequenz verbessern und die mit der Verstopfung verbundenen Symptome lindern.

EP 2620506 B1 (DUPONT) betrifft die Gewinnung von GOS ausgehend von Lactitol.

EP 3598901 B1 (HOCHSCHULE ANHALT) betrifft ein Verfahren zur Herstellung von GOS, bei dem man eine von L.bulgaricus (L.delbrueckii spp.bulgaricus) abgeleitete beta-Galactosidase bei einer Temperatur von 37 C oder weniger mit einer Lactose-haltigen Zusammensetzung wie Milch, Puffer oder Molke, z.B. Süßmolke, Sauermolke, Molkenkonzentrat oder Molkenpermeat, inkubiert.

EP 3041945 B1 (FRIESLAND) stellt ein Verfahren zur Herstellung von GOS aus Lactose bereit, das (i) das Inkontaktbringen einer Lactosebeschickung mit immobilisierter beta-Galactosidase (EC 3.2.1.23) und (ii) das Ermöglichen der GOS-Synthese umfasst, wobei die Lactosebeschickung eine wässrige Aufschlämmung von kristalliner Lactose ist.

WO 2008 037839 A1 (VALIO) bezieht sich auf ein Verfahren zur Herstellung von GOShaltigen Produkten auf Milchbasis durch Behandlung mit einer beta-Galactosidase.

WO 2018 048305 A1 (UNIV GRONINGEN) beschreibt die Verwendung einer GOS-Zusammensetzung, die verzweigte und lineare GOS-Spezies mit einem Polymerisationsgrad (DP) von 3 umfasst, wobei die verzweigten DP3-GOS-Spezies im Überschuss gegenüber den linearen DP3-GOS-Spezies vorhanden sind, zur Induktion von Mucin-Glykan-Verwertungswegen in nützlichen Darmbakterien in einem Tier.

WO 2018 210820 A1 (NOVOZYMES) beansprucht ein Verfahren, bei dem Milchsubstrat mit einem Laktosegehalt von mindestens 20 Gew.-% Laktose wird mit einem Enzym mit transgalaktosylierender Aktivität behandelt wird. Die transgalaktosylierende Aktivität des Enzyms kann durch Glykation von Lysin- und/oder Argininresten durch Inkubation des Enzyms mit hohen Glukosekonzentrationen bei erhöhten Temperaturen erhöht worden sein.

WO 2020 049016 A1 (FRIESLAND) bezieht sich auf das Gebiet der hypoallergenen Oligosaccharide zur Verwendung in Nahrungszusammensetzungen, insbesondere auf Oligosaccharide mit präbiotischen Eigenschaften. Es wird eine hypoallergene Oligosaccharid-Zusammensetzung bereitgestellt, die Galactooligosaccharide (GOS) umfasst, wobei (i) der Gehalt an Galactooligosacchariden (GOS) mindestens 40 Gew.-% der Gesamttrockensubstanz der Zusammensetzung beträgt; (ii) der Gehalt an Allolactose mindestens 10 Gew.-% der Gesamttrockensubstanz der Zusammensetzung beträgt; (iii) der Gehalt an 6'-GL mindestens 30 Gew.-% der Gesamt-GOS in der Zusammensetzung beträgt; und (iv) mindestens 0. 5 Gewichtsprozent des gesamten GOS einen Polymerisationsgrad (DP) von sechs oder mehr aufweisen.

WO 2020 117548 A1 (DUPONT) betrifft ein Verfahren zur Bereitstellung eines laktosearmen Produkts auf Milchbasis mit GOS-Fasern, bei dem ein Milchsubstrat mit Laktose mit einem transgalaktosylierenden Enzym behandelt wird, um GOS-Fasern und verbleibende Laktose bereitzustellen; Deaktivieren des transgalaktosylierenden Enzyms; Inkontaktbringen des Substrats auf Milchbasis mit GOS-Fasern mit einer Laktase, um die verbleibende Laktose abzubauen, um das laktosearme Produkt auf Milchbasis mit GOS-Fasern bereitzustellen, und Deaktivieren der Laktase.

WO 2020 141032 A1 (FRIESLAND) bezieht sich auf das Gebiet der Nahrungsmittelbestandteile, insbesondere auf wirtschaftlich attraktive Verfahren zur Herstellung von hypoallergenen Galactooligosacchariden (HA-GOS) und deren Verwendung in Nahrungs- und Futtermitteln. Es wird ein Verfahren zur Herstellung eines HA-GOS-Präparats bereitgestellt, das das Inkontaktbringen eines Lactose-Einsatzmaterials mit einer speziellen beta-Galactosidase (EC 3.2.1.23) umfasst, wobei das Lactose-Einsatzmaterial ein Käsemolkenpermeat (CWP) oder ein CWP ist, das mit Sialyllactose angereichert ist (SL-CWP).

Lactulose ist ein bekannter Wachstumsfaktor für Bifidobacterium. Ihre Wirksamkeit bei der Aufrechterhaltung der Gesundheit des Menschen ist gut dokumentiert. Außerdem ist die Wirksamkeit von Lactulose nicht auf den Menschen beschränkt, sondern wurde auch für Tierfutter nachgewiesen, was bedeutet, dass ihre Anwendung auf einer Vielzahl von Gebieten untersucht worden ist.

Über die Wirksamkeit von Lactulose beim Menschen wird beispielsweise in *"*The Many Faces of Lactulose: Recent Research Trends in Development and Physiological Effects" (Milk Science, Bd. 50, Nr. 2 (2001), S. 39-47) berichtet, wo Informationen bezüglich der Wachstumsaktivität bei Bifidobacterium gegeben werden. Mittels dieser Aktivität sorgt Lactulose für eine Reihe von Wirkungen, einschließlich einer Verbesserung der Umgebung im Darm, einer Verbesserung der Ausscheidung und einer Beschleunigung der Darmentleerung, was bedeutet, dass die positiven Wirkungen auf die Gesundheit des Menschen bekannt sind.

Das Bewusstsein für eine gesunde Ernährungsweise ist in den letzten Jahren unter Verbrauchern kontinuierlich gestiegen. Die Einnahme von Probiotika ist ein wichtiger Schritt für Menschen, die ihre allgemeine Gesundheit und ihr Wohlbefinden verbessern wollen.

Demzufolge gibt es den Bedarf neue Probiotika dem Verbraucher zur Verfügung zu stellen.

Die grundlegende Idee der vorliegenden Erfindung ist eine neue probiotische Zusammensetzung bereitzustellen, die zwei Substanzen, welche für ihre probiotische Wirkung bekannt sind, enthält.

Die Aufgabe der vorliegenden Erfindung hat daher darin bestanden, Galactooligosaccharide einen hohem Lactulosegehalt zur Verfügung zu stellen. Neben einem hohem Lactulosegehalt, sollen die erfindungsgemäßen Galactooligosaccharide eine verminderte Menge an Lactose und Monosacchariden und niedrigen Oligosacchariden aufweisen.

### BESCHREIBUNG DER ERFINDUNG

Ein erster Gegenstand der Erfindung betrifft Galactooligosaccharide mit einem hohen Lactulosegehalt, dadurch erhältlich oder erhalten, dass man
(a) eine wässrige Zusammensetzung umfassend Lactose und Fructose bereitstellt;
(b) die wässrige Zusammensetzung aus Schritt (a) entkeimt;
(c) den in der entkeimten wässrigen Zusammensetzung aus Schritt (b) vorhandenen Lactose und Fructose unter Zugabe mindestens einer β-Galactosidase innerhalb deren optimalen Temperatur- und pH-Wert-Intervalls über einen Zeitraum von mindestens 30 Minuten einer Transgalactosilierung unter Erhalt einer Reaktionsmischung unterwirft;
(d) die in der Reaktionsmischung aus Schritt (c) enthaltene Enzymmasse inhibiert;
(e) das Zwischenprodukt aus Schritt (d) unter Erhalt eines Retentats R1 umfassend die inhibierten Enzyme und eines Permeates P1 umfassend GOS, Lactulose, Lactose und Monosacchariden und niedrigen Oligosacchariden filtriert;
(f) das Permeat P1 aus Schritt (e) optional mit einer Lactosehydrolase und/oder einer Hefe, die Lactose zu CO2 und Ethanol verstoffwechseln kann, nachbehandelt und anschließend einer Ultrafiltration unter Erhalt eines Retentats R2 und eines Permeates P2 unterworfen;
(g) das Permeat P1 aus Schritt (e) oder das Permeat P2 aus Schritt (f) unter Erhalt eines Retentats R3 und eines Permeates P3 filtiert;
(h) Konfektionierung des Retentats R3 aus Schritt (g)

Ein weiterer Gegenstand der Erfindung betrifft ein Verfahren zur Herstellung von Galactooligosacchariden, insbesondere Galactooligosaccharide mit einem hohen Lactulosegehalt, umfassend oder bestehend aus folgenden Schritten:
(a) Bereitstellen einer wässrigen Zusammensetzung umfassend Lactose und Fructose;
(b) Entkeimung der wässrigen Zusammensetzung aus Schritt (a);
(c) Transgalactosilierung der in der entkeimten wässrigen Zusammensetzung aus Schritt (b) vorhandenen Lactose und Fructose unter Zugabe mindestens einer β-Galactosidase innerhalb deren optimalen Temperatur- und pH-Wert-Intervalls über einen Zeitraum von mindestens 30 Minuten unter Erhalt einer Reaktionsmischung;
(d) Inhibierung der Enzymmasse in der Reaktionsmischung aus Schritt (c);
(e) Filtration des Zwischenprodukts aus Schritt (d) unter Erhalt eines Retentats R1 umfassend die inhibierten Enzyme und eines Permeates P1 umfassend GOS, Lactulose, Lactose und Monosacchariden und niedrigen Oligosacchariden;
(f) optional Nachbehandlung des Permeates P1 aus Schritt (e) mit einer Lactosehydrolase und/oder einer Hefe, die Lactose zu CO2 und Ethanol verstoffwechseln kann und anschließende Ultrafiltration unter Erhalt eines Retentats R2 und eines Permeates P2;
(g) Filtration des Permeates P1 aus Schritt (e) oder des Permeates P2 aus Schritt (f) unter Erhalt eines Retentats R3 und eines Permeates P3;
(h) Konfektionierung des Retentats R3 aus Schritt (g).

In einer besonderen Ausführungsform zeichnet sich das erfindungsgemäße Verfahren dadurch aus, dass man
(i) die Enzymmasse durch Einstellen eines pH-Wertes außerhalb des Aktivitätsoptimums ganz oder teilweise inhibiert; und
(ii) die inhibierte Enzymmenge als Retentat (R1) abtrennt und zum Schritt (c) zurückführt.

Überraschenderweise wurde gefunden, dass Galactooligosaccharidlösungen sich mittels des erfindungsgemäßen Verfahrens herstellen lassen, die einen Lactulosegehalt von mindestens etwa 5 Gew.-% aufweisen, wobei die Menge an Rest-Lactose, Monosacchariden und niedrigen Oligosacchariden unterhalb von 10 Gew.-% liegt.

In Sinne der vorliegenden Erfindung liegt der Lactulosegehalt der erfindungsgemäßen Galactooligosaccharidmischungen bzw. der mittels des erfindungsgemäßen Verfahrens hergestellten Galactooligosacchariden im Bereich von etwa 5 bis 20 Gew.-%, insbesondere von etwa 10 bis 15 Gew.-%.

### Einsatzstoffe

In Sinne der vorliegenden Erfindung kommen als Einsatzstoffe wässrige Zusammensetzungen in Frage, die eine ausreichende Menge an Lactose, speziell an glykosidisch gebundener Galactose besitzen, und Fructose besitzen.

In einer bevorzugten Ausführungsform ist die wässrige Zusammensetzung ausgewählt aus der Gruppe bestehend aus mit Fructose angereicherten Lactoselösung, mit Fructose angereicherten Sauermolke, mit Fructose angereicherten Milchpermeat oder einem Sirup bestehend aus einer Mischung aus Glucose, Galactose und Fructose.

In einer weiteren bevorzugten Ausführungsform weisen die oben genannten wässrigen Zusammensetzungen einen Fructosegehalt von etwa 10 bis 25 Gew.-% auf, insbesondere von etwa 15 bis 25 Gew.-%.

In einer weiteren bevorzugten Ausführungsform weisen die oben genannten wässrigen Zusammensetzungen einen Lactosesegehalt von etwa 20 bis 50 Gew.-% auf, insbesondere von etwa 25 bis 35 Gew.-%.

In einer weiteren bevorzugten Ausführungsform weisen die oben genannten wässrigen Zusammensetzungen einen Lactosesegehalt von etwa 20 bis 50 Gew.-% auf, insbesondere von etwa 25 bis 35 Gew.-% und einen Fructosegehalt von etwa 10 bis 25 Gew.-% auf, insbesondere von etwa 15 bis 25 Gew.-%.

In Falle der Verwendung des Sirups, wird er vorzugsweise vorab hergestellt, und zwar aus Lactose. Vorzugsweise wird dabei Lactose reinst (Pharma-Qualität) verwendet. Sollte man eine Lactose mit weniger Qualität verwendet, kann diese vorab gereinigt werden, beispielweise mittels Kationenaustauscher um Calcium zu entfernen. Unter Verwendung von einer β-Galactosidase wird die Lactose hydrolysiert, sodass ein Glucose-Galactose Sirup erhalten wird. Danach erfolgt die Glucoseisomerisierung, sodass Sirup bestehend aus Glucose (25 Gew.-%), Galactose (50 Gew.-%) und Fructose (25 Gew.-%) erhalten wird. Dieses Verfahren ist für den Fachmann bestens bekannt, und braucht daher nicht in Detail beschrieben werden (siehe Luzzi, G., Steffens, M., Clawin-Rädecker, I., Hoffmann, W., Franz, C.M.A.P., Fritsche, J. and Lorenzen, P.C. (2020), Enhancing the sweetening power of lactose by enzymatic modification in the reformulation of dairy products. Int J Dairy Technol, 73: 502-512). Selbstverständlich kann aus dem Sirup die Fructose chromatographisch isoliert werden, und diese isolierte Fructose kann verwendet werden, um eine wässrige Lactoselösung oder Sauermolke oder ein Milchpermeat mit Fructose anzureichern.

Es empfiehlt sich, wässrige Zusammensetzungen im Schritt (a) mit einer ausreichend hohen Trockenmasse einzusetzen, um das erfindungsgemäße Verfahren mit ökonomisch sinnvollen Umsätzen und Ausbeuten durchführen zu können. Hierzu eignen sich Lösungen, die eine Trockenmasse von etwa 25 bis etwa 50 Gew.-% und vorzugsweise etwa 30 bis etwa 35 Gew.-% aufweisen. Gegebenenfalls können die wässrigen Zusammensetzungen beispielsweise durch Umkehrosmose ("reverse osmosis RO") entsprechend aufkonzentriert werden.

### Entkeimung

Die wässrigen Zusammensetzungen aus Schritt (a) werden entkeimt. Darunter ist jeder Prozess zu verstehen, mit dem sich die Keimlast des Ausgangsproduktes auf einen Wert vermindern lässt, der unter dem liegt, den die jeweiligen nationalen Prüfstellen als Schwelle für die Zulassung als Lebensmittel festgelegt haben. In der Regel werden die wässrigen Zusammensetzungen auf unter 1.000 Keime/mL entkeimt, vorzugsweise auf unter 500 Keime/mL und insbesondere etwa 10 bis etwa 50 Keime/mL. Die bevorzugte Entkeimungsmethode ist eine Hochtemperaturbehandlung, bei der die wässrigen Zusammensetzungen einer Temperatur im Bereich von etwa 70 bis etwa 150 °C, vorzugsweise etwa 90 bis etwa 120 C über etwa 3 bis etwa 300 Sekunden, vorzugsweise etwa 50 bis etwa 200 Sekunden ausgesetzt werden.

### Enzymatische Transgalactosilierung

Die entkeimten Zwischenprodukte werden im Schritt (c) einer enzymatischen Transgalactosilierung unterworfen. Hierunter versteht man die Übertragung von Galactoseeinheiten unter Aufbau eines oligomeren Zuckers in Gegenwart geeigneter Enzyme, in diesem Fall von β-Galactosidasen, wobei Enzyme aus *Aspergillus oryzae, Bacillus circulans* oder Mischungen von beiden gemeinsam bzw. nacheinander zum Einsatz kommen.

*Aspergillus oryzae,* richtiger *Aspergillus flavus* var. *Oryzae* ist ein Schimmelpilz (Gießkannenschimmel), der in der japanischen Küche eine große Rolle spielt. Er ist der wichtigste unter den Köji-Pilzen Er wird vor allem benutzt, um Soja in Feststoff-Bioreaktoren zu fermentieren und so Miso und Sojasauce zu erzeugen.

*Bacillus circulans* ist eine Bakterienspezies, die sich zirkulär auf Nährmedien ausbreitet, woher auch ihr Namen stammt. Es handelt sich um anaerob wachsende, Gram variable stäbchenförmige, bewegliche Zellen, die 0,5 bis 1 µm breit und 3,5 µm lang sind. Das Bakterium fermentiert Pentosen, Hexosen, Hexitole und Disaccharide. *Bacillus circulans* kommt im Darm von pflanzenfressenden Fischen vor und unterstützt dort durch die Ausscheidung von Cellulasen die Verdauung.

Wie alle Enzyme weisen auch die β-Galactosidasen ein vergleichsweise enges Temperatur- und pH-Wertintervall auf, in dem sie ihre optimale Leistung entfalten; diese sind dem Fachmann notorisch bekannt.

Mit *Aspergillus oryzae* wird die Reaktion daher vorzugsweise bei einer Temperatur im Bereich von etwa 50 bis etwa 60 °C und einem pH-Wert von etwa 4 bis etwa 5, bei Zugabe von Enzymen aus *Bacillus circulans* bei einer Temperatur im Bereich von etwa 45 bis etwa 55 °C und einem pH-Wert von etwa 5,5 bis etwa 6,5 durchführt. Bei Einsatz von *Aspergillus oryzae* wird daher auch von einem *"sauren Verfahren",* bei *Bacillus circulans* von einem *"neutralen Verfahren"* gesprochen.

Eine Besonderheit bei der grundsätzlichen Bildung von Galactooligosacchariden besteht darin, dass sich der Kettenaufbau nicht stetig fortsetzt, sondern nach einer Zeit an Geschwindigkeit verliert, bis sogar die Konkurrenzreaktion, nämlich die Rückspaltung der GOS überwiegt.

Es hat sich daher als vorteilhaft erwiesen, der enzymabhängigen Reaktionskinetik Rechnung zu tragen und die Transgalactosilierung über einen Zeitraum von etwa 30 bis etwa 1200 min und insbesondere von etwa 60 bis etwa 90 min durchzuführen. Dabei gilt, dass hohe initiale Lactosekonzentrationen die Hydrolyseneigung deutlich vermindern und niedrige Enzymkonzentration die Hydrolyse ebenfalls verzögern.

### Inhibierung: pH-Shift

Die Transgalactosilierung wird vorzugsweise solange durchgeführt, bis die höchste GOS-Konzentration erreicht ist. Dieser durch Enzym und Reaktionsbedingungen bedingte Wert kann durch Probennahme verfolgt und damit für den Fachmann leicht ermittelt werden. Ist das Maximum der GOS-Bildung erreicht, muss die Aktivität der Enzyme sehr schnell gestoppt werden, um eine Rückspaltung zu vermeiden. Dies kann beispielsweise durch schnelle Hocherhitzung erfolgen, bei der allerdings das Enzymmaterial vollständig abgetötet wird. Die vorliegende Erfindung bevorzugt einen anderen Weg und führt die Enzyme aus ihren optimalen Reaktionsbedingungen, konkret wird der pH-Wert durch Zugabe von Basen gegenüber dem Optimum um mindestens zwei Einheiten heraufgesetzt oder durch Zugabe von Säuren um mindestens zwei Einheiten herabgesetzt. Dieser pH-Shift bringt die Reaktion zwar nicht schlagartig zum Erliegen, vermindert die Aktivität der Enzyme jedoch um 80 bis 90 %, was für die Anforderungen ausreicht, eine nennenswerte Rückspaltung zu verhindern. Hierzu reicht es, den pH auf Werte von mindestens 7, vorzugsweise 8 bis 12 und insbesondere 9 bis 10 zu erhöhen oder auf Werte von 2 bis 5, vorzugsweise 3 bis 4 herabzusetzen.

Bei der Änderung des pH-Wertes ist zu berücksichtigen, dass die Enzyme nicht irreversibel inaktiviert werden und der entsprechende pH-Wert - speziell im sauren Bereich - eine spätere Nutzung des Produktes nicht behindert. Der pH-Shift kann durch Zugabe einer erforderlichen Menge üblicher anorganischer Basen, wie beispielsweise einer wässrigen NaOH, durch Mineralsäuren wie HCl oder organische Säuren, wie beispielsweise Milchsäure erfolgen. Eine Anhebung des pH-Wertes ist gegenüber einer Absenkung bevorzugt.

### Abtrennung und Wiederverwendung der Enzyme

Die Abtrennung der Enzymmasse erfolgt vorzugsweise durch Filtration und insbesondere durch Ultrafiltration, die ebenfalls vorzugsweise kontinuierlich durchgeführt wird. Dabei wird ein Retentats R1 umfassend die inhibierten Enzyme und eines Permeates P1 umfassend GOS, Lactulose, Lactose und Monosacchariden und niedrigen Oligosacchariden erhalten.

Die abgetrennte Enzymmenge wird wieder in den Reaktionskreislauf - im Schritt (c) - zurückgeführt. Dabei gelangen die Enzyme direkt wieder in den Temperatur- und pH-Wertbereich, in dem ihr Optimum sich befindet. Gegebenenfalls kann eine solche Menge frisches Enzym nachdosiert werden, so dass die Enzymaktivität über das kontinuierliche Verfahren hinweg konstant oder wenigstens annähernd konstant bleibt.

Wie bereits erwähnt, erfolgt die Abtrennung der Enzymmasse vorzugsweise mittels Ultrafiltration.

In einer bevorzugten Ausführungsform erfolgt die Ultrafiltration bei Temperaturen im Bereich von etwa 10 bis etwa 55, vorzugsweise 10 bis 20 °C, wobei die Membranen vorzugsweise einen Porendurchmesser im Bereich von etwa 1 bis etwa 50 kDa und vorzugsweise etwa 5 bis etwa 25 kDa besitzen. Vorzugsweise handelt es sich um sogenannte Spiralwickelmembranen oder Platte-Rahmen-Module aus Polysulfon oder Polyethylenmembranen.

### Optionale Nachbehandlung

Optional, kann das im Schritt (e) gewonnene Permeat P1 einer Nachbehandlung unterworfen werden, und zwar durch versetzen des Permeats P1 mit Lactosehydrolase und/oder einer Hefe, die die Reste von Lactose zu CO₂ und Ethanol verstoffwechseln kann.

Vorzugsweise wird als Hefe *Kluyvermomyces lactis* eingesetzt. Auch die Nachbehandlung wird innerhalb deren optimalen Temperatur- und pH-Wert-Intervalls der Hydrolase und/oder Hefe - in der Regel zwischen 28 und 37 C - über einen Zeitraum von mindestens 30 Minuten durchgeführt. Eine Inhibierung der Hydrolasen und/oder Hefen ist in diesem Fall nicht zwingend erforderlich, kann aber je nach weiterem Einsatzzweck Sinn machen.

Anschließend erfolgt eine zweite Ultrafiltration, die zur Abtrennung der Enzym- bzw. Enzym/Hefemasse dient. Gewonnen werden ein Retentat R2, welches Enzym- bzw. Enzym/Hefemasse enthält, und ein Permeat P2, welches unter anderen GOS und Lactulose enthält.

In einer bevorzugten Ausführungsform erfolgt die Ultrafiltration bei Temperaturen im Bereich von etwa 10 bis etwa 55, vorzugsweise 10 bis 20 °C, wobei die Membranen vorzugsweise einen Porendurchmesser im Bereich von etwa 1 bis etwa 50 kDa und vorzugsweise etwa 5 bis etwa 25 kDa besitzen. Vorzugsweise handelt es sich um sogenannte Spiralwickelmembranen oder Platte-Rahmen-Module aus Polysulfon oder Polyethylenmembranen.

### 2. Filtration und Konfektionierung (Aufreinigung, Konzentrierung und Trocknung)

Das Permeat P1 aus dem Schritt (e) bzw. das Permeat P2 aus Schritt (f) wird einer Filtration unterworfen, sodass ein Retentat R3, welches GOS und Lactulose enthält, und ein Permeat P3, welche unerwünschten Nebenprodukte wie Monosaccharide, Reste von Lactose und niedrigen Oligosacchriden enthält, erhalten werden.

In einer bevorzugte Ausführungsform erfolgt die Filtration des Schrittes (g) mittels Nanofiltration.

In einer weiteren bevorzugten Ausführungsform erfolgt die Nanofiltration bei Temperaturen im Bereich von etwa 6 bis etwa 60, vorzugsweise 6 bis 20 °C, wobei die Membranen vorzugsweise einen Porendurchmesser im Bereich von etwa 0,1 bis etwa 2 kDa und vorzugsweise etwa 0,5 bis etwa 1 kDa besitzen. Vorzugsweise handelt es sich um sogenannte Spiralwickelmembranen aus Polymerwerkstoffen oder Kerzenfilter aus Keramik oder Aluminiumoxid.

Um konfektionierfähige Produkte zu erhalten, wird das Retentats R3 getrocknet und zuvor gegebenenfalls aufgereinigt und/oder aufkonzentriert.

Um die GOS-Konzentration zu erhöhen kann das Retentat R3 noch aufgereinigt werden, beispielsweise durch Elektrodialyse oder Membranverfahren, wie z.B. eine Umkehrosmose. Falls erforderlich, kann die Trockenmasse durch Eindampfen erhöht werden

Die Trocknung erfolgt beispielsweise durch Lyophilisierung, vorzugsweise aber durch Sprühtrocknung, wobei die Temperatur im Einlass typischerweise etwa 180 bis etwa 260 °C und am Ausgang etwa 80 bis etwa 105 °C beträgt. Der Restwassergehalt beträgt dabei maximal 5 Gew.-% und vorzugsweise etwa 1 bis etwa 2 Gew.-%.

Ein weiterer Gegenstand der Erfindung betrifft die Verwendung der neuen Galactooligosaccharide sowie der Stoffe, die nach dem erfindungsgemäßen Verfahren erhalten werden, als Nahrungsmittelergänzungsstoffe sowie Produkte für die Tierernährung.

### BEISPIELE

Die vorliegende Erfindung wird unter Bezugnahme auf die nachstehenden Beispiele leichter zu verstehen sein.

Jedoch dienen diese Beispiele lediglich zur Veranschaulichung der Erfindung und können nicht als einschränkend in Bezug auf den Schutzbereich der Erfindung ausgelegt werden.

### BEISPIEL 1: Herstellung von GOS nach dem neutralen Verfahren ausgehend von einer mit Fructose angereicherten Lactoselösung

1.000 kg einer wässrigen Lactose-Fructose-Lösung (30 Gew.-% Lactose, 18 Gew.-% Fructose) wurde in einem Röhrenwärmeaustauscher über 120 Sekunden auf 98 C erhitzt und dabei entkeimt.

Die entkeimte Lösung wurde auf 55 C abgekühlt, in einen Fermenter überführt, mit Hilfe von Milchsäure auf einen pH-Wert von 6,5 eingestellt, mit β-Galactosidase *aus Bacillus circulans* im Gewichtsverhältnis Enzym:Substrat von 1:50 versetzt und gerührt. Der Fortschritt der Transgalactosilierung wurde durch Probennahme verfolgt. Nach etwa 90 Minuten war die maximale GOS-Konzentration erreicht.

Der pH-Wert wurde durch Zugabe von 30 Gew.-%iger Natronlauge innerhalb von wenigen Minuten auf 10 erhöht, wodurch die Aktivität des Enzyms schlagartig um 80 % verringert wurde.

Der Reaktionsansatz wurde zu einer Ultrafiltrationseinheit geleitet, die mit einer Spiralwickelmembran mit einer Porengröße von 10 kDa versehen war. Das inaktivierte Enzymmaterial wurde mit dem Retentat R1 in den Fermenter zurückgeführt; um Verlust auszugleichen wurden bezogen auf die Ausgangsmenge 5 Gew.-% frisches Enzym zugefügt.

Das Permeat P1 wurde mit Lactosehydrolase im Gewichtsverhältnis Enzym/Hefe:Substrat von 1:25 und bei 35 C etwa 10 Stunden gerührt.

Der Reaktionsansatz wurde zu einer zweiten Ultrafiltrationseinheit geleitet, die mit ebenfalls mit einer Spiralwickelmembran mit einer Porengröße von 10 kDa versehen war.

Die Enzym/Hefemasse wurde mit dem Retentat R2 abgetrennt und das Permeat P2 zu einer Nanofiltrationseinheit geleitet, die mit einer Keramikmembran mit einer Porenweite von 500 bis 1.000 Da versehen war, unter Erhalt eines Permeates P3 und eines Retentates R3.

Mit dem Permeat P3 wurden die noch im Produkt enthaltenen Monosaccharide abgetrennt, während das Retentat R3 einer Umkehrosmoseeinheit zugeleitet wurde, die mit einem Konzentrierungsfaktor von 1:2 arbeitete. Das dabei anfallende Permeat P4 (also das Konzentrierungswasser) wurde in das Verfahren zurückgeleitet, das Retentat R4 (also das mit Lactulose eingereichte GOS-Konzentrat) wurde im Plattenwärmeaustauscher 30 Sekunden auf etwa 85 C erhitzt und über einen Turm versprüht.

Es wurde ein weißes Pulver mit einem GOS-Gehalt von mehr als 75 Gew.-% und einem Lactulose-Gehalt von14 Gew.-% erhalten, welches noch eine Restfeuchte von 1 Gew.-% und einen Monosaccharidgehalt von 0,4 Gew.-% aufwies.

## Patentansprüche

1. Galactooligosaccharide mit einem hohen Lactulosegehalt, dadurch erhältlich oder erhalten, dass man
(a) eine wässrige Zusammensetzung umfassend Lactose und Fructose bereitstellt;
(b) die wässrige Zusammensetzung aus Schritt (a) entkeimt;
(c) den in der entkeimten wässrigen Zusammensetzung aus Schritt (b) vorhandenen Lactose und Fructose unter Zugabe mindestens einer β-Galactosidase innerhalb deren optimalen Temperatur- und pH-Wert-Intervalls über einen Zeitraum von mindestens 30 Minuten einer Transgalactosilierung unter Erhalt einer Reaktionsmischung unterwirft;
(d) die in der Reaktionsmischung aus Schritt (c) enthaltene Enzymmasse inhibiert;
(e) das Zwischenprodukt aus Schritt (d) unter Erhalt eines Retentats R1 umfassend die inhibierten Enzyme und eines Permeates P1 umfassend GOS, Lactulose, Lactose und Monosacchariden und niedrigen Oligosacchariden filtriert;
(f) das Permeat P1 aus Schritt (e) optional mit einer Lactosehydrolase und/oder einer Hefe, die Lactose zu CO2 und Ethanol verstoffwechseln kann, nachbehandelt und anschließend einer Ultrafiltration unter Erhalt eines Retentats R2 und eines Permeates P2 unterworfen;
(g) das Permeat P1 aus Schritt (e) oder das Permeat P2 aus Schritt (f) unter Erhalt eines Retentats R3 und eines Permeates P3 filtiert;
(h) Konfektionierung des Retentats R3 aus Schritt (g)

2. Verfahren zur Herstellung von Galactooligosacchariden, insbesondere Galactooligosaccharide mit einem hohen Lactulosegehalt, umfassend oder bestehend aus folgenden Schritte:
(a) Bereitstellen einer wässrigen Zusammensetzung umfassend Lactose und Fructose;
(b) Entkeimung der wässrigen Zusammensetzung aus Schritt (a);
(c) Transgalactosilierung der in der entkeimten wässrigen Zusammensetzung aus Schritt (b) vorhandenen Lactose und Fructose unter Zugabe mindestens einer β-Galactosidase innerhalb deren optimalen Temperatur- und pH-Wert-Intervalls über einen Zeitraum von mindestens 30 Minuten unter Erhalt einer Reaktionsmischung;
(d) Inhibierung der Enzymmasse in der Reaktionsmischung aus Schritt (c);
(e) Filtration des Zwischenprodukts aus Schritt (d) unter Erhalt eines Retentats R1 umfassend die inhibierten Enzyme und eines Permeates P1 umfassend GOS, Lactulose, Lactose und Monosacchariden und niedrigen Oligosacchariden;
(f) optional Nachbehandlung des Permeates P1 aus Schritt (e) mit einer Lactosehydrolase und/oder einer Hefe, die Lactose zu CO2 und Ethanol verstoffwechseln kann und anschließende Ultrafiltration unter Erhalt eines Retentats R2 und eines Permeates P2;
(g) Filtration des Permeates P1 aus Schritt (e) oder des Permeates P2 aus Schritt (f) unter Erhalt eines Retentats R3 und eines Permeates P3;
(h) Konfektionierung des Retentats R3 aus Schritt (g).

3. Verfahren nach Anspruch 2, **dadurch gekennzeichnet, dass** man
(i) die Enzymmasse durch Einstellen eines pH-Wertes außerhalb des Aktivitätsoptimums ganz oder teilweise inhibiert; und
(ii) die inhibierte Enzymmenge als Retentat (R1) abtrennt und zum Schritt (c) zurückführt.

4. Verfahren nach einem der vorhergehenden Ansprüche, **dadurch gekennzeichnet, dass** die wässrige Zusammensetzung im Schritt (a) aus der Gruppe bestehend aus mit Fructose angereicherten Lactoselösung, mit Fructose angereicherten Sauermolke, mit Fructose angereicherten Milchpermeat oder einem Sirup bestehend aus einer Mischung aus Glucose, Galactose und Fructose ausgewählt ist.

5. Verfahren nach einem der vorhergehenden Ansprüche, **dadurch gekennzeichnet, dass** man im Schritt (a) wässrige Zusammensetzungen einsetzt, die eine Trockenmasse von etwa 25 bis etwa 50 Gew.-% aufweisen.

6. Verfahren nach einem der vorhergehenden Ansprüche, **dadurch gekennzeichnet, dass** man die Entkeimung durch eine Hochtemperaturbehandlung bewirkt.

7. Verfahren nach einem der vorhergehenden Ansprüche, **dadurch gekennzeichnet, dass** man als β-Galactosidase Enzyme aus *Aspergillus oryzae* und/oder *Bacillus circulans* einsetzt.

8. Verfahren nach Anspruch 7, **dadurch gekennzeichnet, dass** man die Transgalactosilierung durch Zugabe von β-Galactosidase aus *Aspergillus oryzae* bei einer Temperatur im Bereich von etwa 50 bis etwa 60 °C und einem pH-Wert von etwa 4 bis etwa 5 durchführt.

9. Verfahren nach Anspruch 7, **dadurch gekennzeichnet, dass** man die Transgalactosilierung durch Zugabe von β-Galactosidase aus *Bacillus circulans* bei einer Temperatur im Bereich von etwa 45 bis etwa 55 °C und einem pH-Wert von etwa 5,5 bis etwa 6,5 durchführt.

10. Verfahren nach einem der vorhergehenden Ansprüche, **dadurch gekennzeichnet, dass** man die Transgalactosilierung im Schritt (c) über einen Zeitraum von etwa 30 bis etwa 1200 min durchführt.

11. Verfahren nach einem der vorhergehenden Ansprüche, **dadurch gekennzeichnet, dass** die Filtration des Schrittes (e) eine Ultrafiltration ist.

12. Verfahren nach einem der vorhergehenden Ansprüche, **dadurch gekennzeichnet, dass** man die Nachbehandlung innerhalb deren optimalen Temperatur- und pH-Wert-Intervalls der Hydrolase und/oder Hefe über einen Zeitraum von mindestens 30 Minuten durchführt.

13. Verfahren nach einem der vorhergehenden Ansprüche, **dadurch gekennzeichnet, dass** die Filtration des Schrittes (g) eine Nanofiltration ist.

14. Verfahren nach einem der vorhergehenden Ansprüche, **dadurch gekennzeichnet, dass** man das Retentats R3 aufkonzentriert und entwässert.

15. Verwendung der Galactooligosaccharide nach Anspruch 1 oder erhalten nach dem Verfahren gemäß den Ansprüchen 2 bis 14 als Nahrungsmittelergänzungsstoffe und Produkte für die Tierernährung.
